(19)

Europäisches
Patentamt
European
Patent Office
Office européen
des brevets

(11)　**EP 3 654 288 A1**

(12)　　　　　　　　　　**EUROPEAN PATENT APPLICATION**

(43) Date of publication:
**20.05.2020　Bulletin 2020/21**

(51) Int Cl.:
**G06T 7/73** *(2017.01)*　　　　**G01N 21/88** *(2006.01)*
**G01N 33/02** *(2006.01)*　　　　*G01N 21/84* *(2006.01)*

(21) Application number: **19209590.9**

(22) Date of filing: **15.11.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:　**15.11.2018　IT 201800010371**

(71) Applicant: **E.P.F. Elettrotecnica S.r.l.**
**12061 Carru' (CN) (IT)**

(72) Inventor: **FILIPPI, Franco**
**12061 CARRU' (CN) (IT)**

(74) Representative: **Spalla, Pietro et al**
**Studio Torta S.p.A.**
**Via Viotti, 9**
**10121 Torino (IT)**

(54)　**SYSTEM FOR DETERMINING THE ORIENTATION OF FRUIT OR VEGETABLE PRODUCTS AND
ENCOMPASSING SYSTEM FOR HANDLING FRUIT OR VEGETABLE PRODUCTS THE SAME**

(57)　　A system for determining the orientation of a fruit or vegetable product, including: a number of capture devices (6,8) that frame the fruit or vegetable product and generate corresponding data structures (SD1,SD2), each of which is indicative of the positions of a corresponding plurality of points; a first processing stage (10) that determines, based on the data structures, the centre (Cc) of a three-dimensional geometric shape that approximates a first surface that depends on the positions of the points; a second processing stage (10) that determines an initial image (Ip2) formed by pixels, which are indicative of the distances from a reference plane (xy) of corresponding portions of a second surface that depends on the first surface; a third processing stage (10) that implements a neural network that detects, within the initial image, the presence and the position of a first and a second set of pixels (GP1, GP2), which form the images of a first and a second characteristic element of the fruit or vegetable product; a fourth stage that calculates a pair of angles, based on the positions of the centre of the three-dimensional geometric shape and of one of either the first or the second set of pixels.

**FIG. 1**

EP 3 654 288 A1

**Description**

CROSS-REFERENCE TO RELATED APPLICATIONS

This patent application claims priority from Italian patent application no. 102018000010371 filed on 15/11/2018.

TECHNICAL FIELD

The present invention relates to a system for determining the orientation of fruit or vegetable products, as well as a system for handling fruit or vegetable products including the same.

BACKGROUND ART

As is known, there are nowadays numerous systems for handling fruit or vegetable products (for example, fruits), which include automatically controlled mechanical arms, which are suitable for picking up the fruit or vegetable products individually and moving them in space, for example for harvesting or packaging purposes. Some of the current handling systems include subsystems suitable to identify fruit or vegetable products from images, so as to be able to operate the corresponding mechanical arms accordingly, minimizing the need for intervention by an operator.

Having said that, the Applicant noted that the current automatic systems for identifying fruit or vegetable products at most allow the position of a fruit or vegetable product to be determined, but not its orientation, unless resorting to roto-translations of the fruit dedicated to this purpose, thus lengthening the cycle times of the packaging operations and risking to damage the product. The consequent actuation of (for example) mechanical arms therefore takes place on the basis of information that may not be sufficiently accurate, in particular when the handling of the fruit or vegetable product must also take into account its orientation, or it may bear the risk of damaging the fruit or vegetable products.

Articles covering automatic harvesting systems include: Zhang Baohua et al., "Computer vision recognition of stem and calyx in apples using near-infrared linear-array structured light and 3D reconstruction", Biosystems engineering, Academic Press, UK, vol. 139, 2015 August 22, pp. 25-34; Peteris Eizentals, "Picking system for automatic harvesting of sweet pepper", 2016 September 1, Kochi, Japan (http://hdl.handle.net/10173/1417); Weilin Wang et al., "Size estimation of sweet onions using consumer-grade RGB-depth sensor", Journal of food engineering, vol. 142, December 2014, pp. 153-162.

DISCLOSURE OF INVENTION

Therefore, the object of the present invention is to provide a system for determining the orientation of a fruit or vegetable product, so as to at least partially overcome the drawbacks of the prior art.

According to the present invention, a system for determining the orientation of a fruit or vegetable product is provided as claimed in the appended claims.

BRIEF DESCRIPTION OF THE DRAWINGS

For a better understanding of the present invention, preferred embodiments thereof will now be described, purely by way of non-limiting example, with reference to the accompanying drawings, wherein:

- Figure 1 schematically shows a perspective view of a portion of a system for handling fruit or vegetable products;
- Figures 2 and 7 show block diagrams relating to operations carried out by the present system;
- Figure 3 shows a block diagram schematically illustrating the dependencies between data structures and quantities determined by the present system;
- Figures 4A and 4B show two-dimensional representations of data contained in data structures;
- Figure 5 shows a two-dimensional representation of data contained in an additional data structure;
- Figures 6 and 8A-8B show images generated by the present system; and
- Figure 9 shows a view of a spherical mathematical model and of two points determined by the present system.

BEST MODE FOR CARRYING OUT THE INVENTION

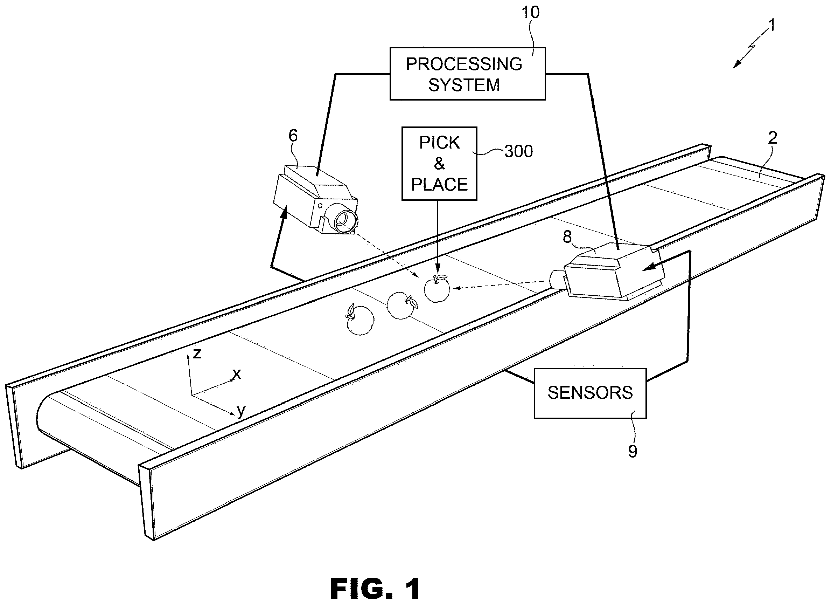

Figure 1 shows a portion of a handling system 1 for handling fruit or vegetable products, which is described below, for simplicity and without any loss of generality, referring to when the fruit or vegetable products are navel-type fruit or vegetable products, i.e. they have a calyx; in particular, reference will be made below to navel-type fruit (apples, for example).

The handling system 1 comprises, purely by way of example, a linear-motion conveyor belt 2. Furthermore, although not shown, the handling system 1 may comprise a so-called singulation system, which is suitable for picking up the fruits from a container and arranging them individually on the conveyor belt 2. Consequently, in a way known per se, on the conveyor belt 2 the fruits are arranged spaced apart, approximately in a line, one behind the other.

The handling system 1 further comprises a first and a second video camera 6, 8, the visual fields of which (sensing ranges) are at least partially superimposed, so that, for each fruit, they respectively provide, as described below, a first and a second data set SD1, SD2, which are referred to below respectively as the first and second data structures SD1, SD2.

The first and second data structures SD1, SD2 refer to the same time instant. In order to time the capture of the first and second data structures SD1, SD2 by the first and second video cameras 6, 8, the handling system 1 may comprise one or more sensors (generally indicated by 9), which are suitable to detect the presence of the fruit inside a portion of space that falls within the visual fields of the first and second video cameras 6, 8. The timing of the capture of the first and second data structures SD1, SD2 is controlled by software algorithms and may be supplemented with information from the sensors 9, which are operatively coupled to the conveyor belt 2.

In greater detail, the first and second video cameras 6, 8 are formed by corresponding profilometers (or stereo cameras), i.e. laser scanning devices, which scan the fruit from two different angles; consequently, each of the aforementioned first and second data structures SD1, SD2 is indicative of the three-dimensional profile detected by the corresponding profilometer. In other words, each of the first and second data structures SD1, SD2 is formed by a plurality of data triplets, each triplet being indicative of the position of a corresponding point of a physical surface (in particular, the surface of the fruit) detected by the profilometer that generated the data structure.

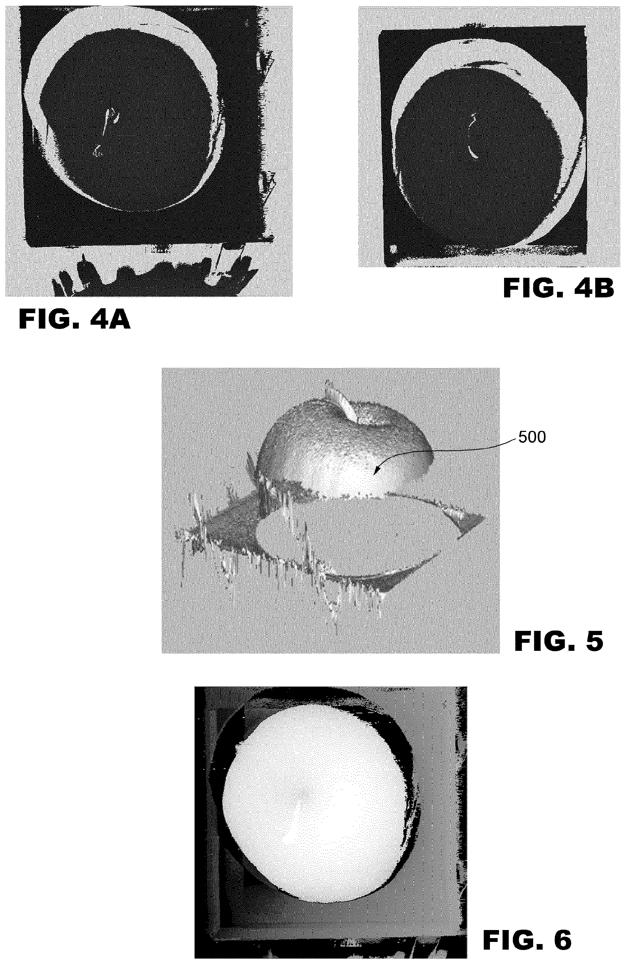

Still in greater detail, Figure 1 shows an orthogonal reference system xyz, whose xy axes lie in the plane of the conveyor belt 2, the x axis being parallel to the direction of movement of the fruits along the conveyor belt 2. Having said that, each of the aforementioned data triplets of the first and second data structures SD1, SD2 represents the coordinates of the corresponding physical point with respect to the aforementioned orthogonal reference system. In this regard, Figures 4A and 4B show two-dimensional representations of examples of a first and a second data structure, such two-dimensional representations being obtained by associating the pixels with shades of grey which depend on the heights of the corresponding points.

Again with reference to Figure 1, it also shows a processing system 10, which is coupled to the first and second video cameras 6, 8, so as to receive the data structures generated by them.

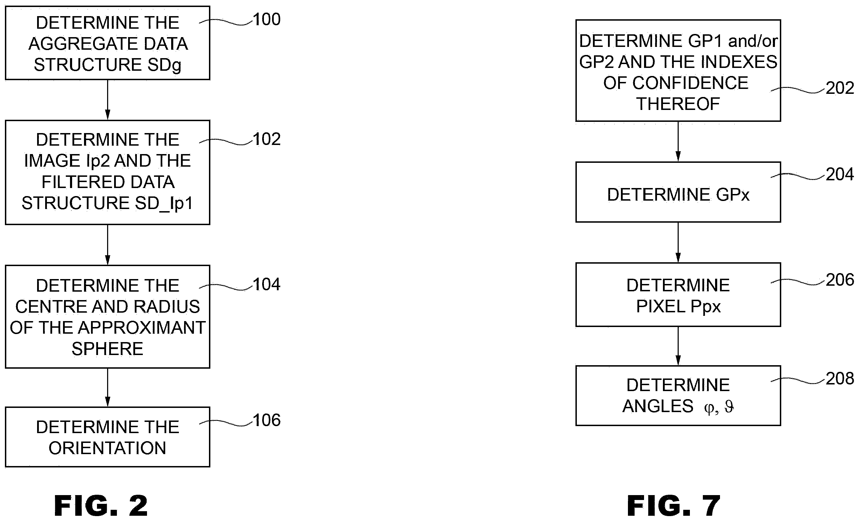

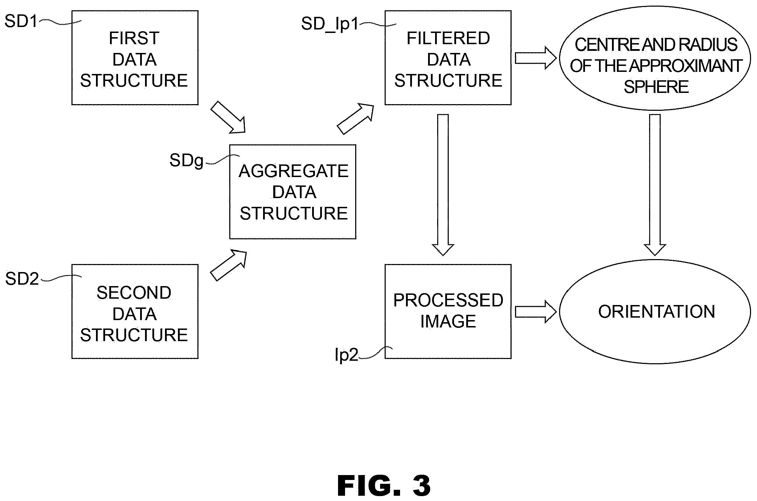

For each pair formed by a first data structure SD1 and by the corresponding second data structure SD2, the processing system 10 performs the operations shown in Figures 2 and 3.

In detail, the processing system 10 determines (block 100, Figure 2) an aggregate data structure SDg, on the basis of the first and second data structures SD1, SD2.

Purely by way of example, the aggregate data structure SDg is formed by the set of data triplets of the first and second data structures SD1, SD2. As previously mentioned, each triplet contains the height value of the corresponding point, this height being measured parallel to the z axis and referring to a reference plane, which coincides, without any loss of generality, with the plane of the conveyor belt 2.

Figure 5 shows a perspective representation of the points contained in the aggregate data structure SDg. It can be seen that the set of points defines a surface 500, which is a function of at least part of the profile of the fruit, and in particular of the upper part of the profile of the fruit, opposite to the part that contacts the conveyor belt 2.

Starting from the aggregate data structure SDg, the processing system 10 determines (block 102, Figure 2) a set of data, which in the following is referred to as the filtered data structure SD_Ip1, and an image Ip2, which in the following is referred to as the processed image Ip2.

The filtered data structure SD_Ip1 is obtained by selecting the points of the aggregate data structure SDg relating to the portion of space in which the fruit is present, so as to discard the spurious points, for example relating to the surrounding environment or caused by erroneous detections, and applying a subsequent spatial filtering (optional). For example, the points that fall within a portion of space having a parallelepiped shape are selected; furthermore, the filtering is such that the value of each point lying in this portion of space is replaced by the barycentre of the set of points formed by the aforesaid point and by a predefined set of adjacent points. In this way, the positions of the points of the filtered data structure SD_Ip1 define a surface, which is less noisy than the surface 500 and has a more limited domain, which in the following is referred to as the filtered surface.

The selection of the aforementioned portion of space of the aggregate data structure SDg is carried out by an operator or takes place automatically; in the latter case, the processing system 10, for example, may search the spatial regions in which there are variations in the height of the points compatible with the typical profile of the fruits and select the aforementioned portion of space so that it contains these regions.

The processed image Ip2, an example of which is shown in Figure 6, is obtained from the data of the filtered data structure SD_Ip1. In particular, the processed image Ip2 is a greyscale map formed by a plurality of pixels and coded, without any loss of generality, according to eight levels of grey; in other words, the pixel values are discretized to eight levels. The data of the filtered data structure SD_Ip1 may therefore have greater precision than the precision allowed by the discretization adopted for the pixels of the processed image Ip2.

In greater detail, the value, and therefore the colour, of each pixel of the processed image Ip2 may be equal, for example, to the discretized level where the average of the heights of the points of the filtered data structure SD_Ip1 which have coordinates along x and y such that the orthogonal projection of these points on the xy plane falls in a region corresponding to the considered pixel falls. In other words, each pixel is indicative of the height of a corresponding portion of the filtered surface defined by the points of the filtered data structure SD_Ip1.

Based on the filtered data structure SD_Ip1, the processing system 10 determines (block 104, Figure 2) the three-dimensional coordinates (Xc,Yc,Zc) of the centre Cc of the sphere (or spherical portion, but for the sake of simplicity reference will be made in the following to the case of the sphere) that best approximates ("fits") the volume of the fruit, i.e. the volume defined by the points of the filtered data structure SD_Ip1, and more precisely that best approximates the filtered surface defined by the points of the filtered data structure SD_Ip1. The aforementioned sphere is determined by applying any one of a number of known fitting algorithms (for example, the least squares method). During the operations referred to in block 104, the processing system 10 also determines the radius of the aforementioned sphere.

Furthermore, starting from the processed image Ip2, the processing system 10 determines (block 106, Figure 2) the orientation of the fruit by performing the operations shown in Figure 7.

In detail, the processing system 10 detects (block 202), within the processed image Ip2, the presence of a first and/or a second set of pixels GP1, GP2, by using a previously trained neural network.

In detail, the neural network was previously trained on the basis of fruit images, so that the first and second sets of pixels GP1 and GP2 form an image of the stem of the fruit and an image of the calyx of the fruit, respectively. Depending on the orientation of the fruit, as previously mentioned, it is possible that only one of the first and second sets of pixels GP1 and GP2 is identified by the neural network, or that neither one is identified; in the latter case, it is possible that the corresponding processed image Ip2 is used to refine the training of the neural network. In the following, however, reference is made to the case in which at least one of the first and second sets of pixels GP1, GP2 is identified by the processing system 10, by implementing the neural network. Furthermore, it is assumed that the neural network is able to associate each of the first and second sets of pixels GP1, GP2 with a corresponding index of confidence, i.e. an index of the probability that the considered set effectively forms the image of a stem (in the case of the first set of pixels GP1) or of a calyx (in the case of the second set of pixels GP2).

In greater detail, each of the first and second sets of pixels GP1, GP2 is formed by a predetermined number of adjacent pixels (for example, a square of 100x100 pixels). Still in greater detail, the dimensions of the first and second sets of pixels GP1, GP2 depend on the previous training of the neural network. In this regard, the training may involve providing the neural network with known fruit images, each of these images showing the stem and/or calyx of the corresponding fruit; in addition, for each known image, the neural network is provided with an indication relating to the position of the portion of the image representing the stem/calyx.

That being said, during the execution of the operations referred to in block 202, the processing system 10 may input to the neural network all the possible portions of the processed image Ip2 having the aforementioned dimensions (for example, 100x100 pixels), for example obtained through the use of a sliding window, so as to perform a pattern recognition on each of these portions, associating each of these portions with a corresponding index of confidence. Alternatively, in order to reduce computational complexity, the processing system 10 may implement an exploration algorithm, for example a hierarchical algorithm, which allows only a subset of all possible portions of the processed image Ip2 to be input to the neural network. In other words, it is possible to implement a criterion for the search of a global or local maximum, which has as its objective function the index of confidence of the two sets of pixels GP1 and GP2 and as search parameters the coordinates in the plane of the test sliding window.

Subsequently, the processing system 10 determines (block 204) a set of reference pixels GPx, which, if the neural network has identified only one of the first and the second set of pixels GP1, GP2, is equal to the identified set of pixels; instead, if the neural network has identified both the first and the second set of pixels GP1, GP2, the set of reference pixels GPx is equal to the set of pixels with which the highest index of confidence is associated.

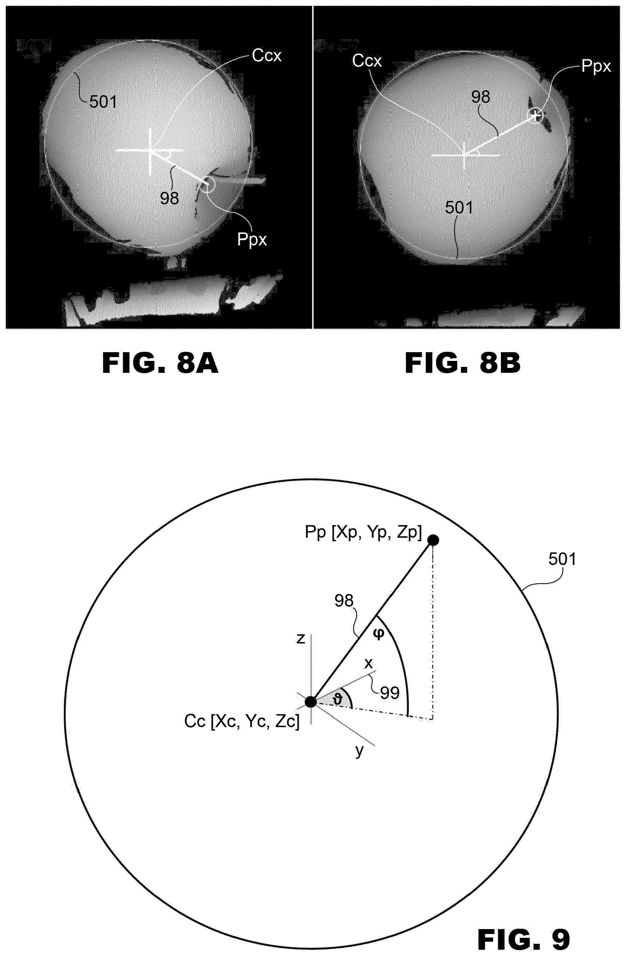

Subsequently, the processing system 10 determines (block 206), within the set of reference pixels GPx, the position of one pixel, which is referred to in the following as the main pixel Ppx. In detail, the main pixel Ppx is a pixel arranged in a predefined position of the set of reference pixels GPx (for example, in the centre) and corresponds to a main point Pp (Figure 9) which has the coordinates (Xp,Yp,Zp), Xp and Yp coinciding with the corresponding coordinates of the main pixel Ppx and with Zp which is calculated by the processing system 10 by using trigonometric relations, based on the coordinates (Xp,Yp) and the radius of the approximant sphere (indicated by R). In particular, with respect to the coordinate system lying on the conveyor belt 2:

$$r = \sqrt{(Xp - Xc)^2 + (Yp - Yc)^2}$$

$$Zp = \sqrt{R^2 - r^2} + R$$

Figures 8A and 8B show two examples of arrangements of the main pixel Ppx, respectively, in the case where the set of reference pixels GPx is equal to the corresponding first set of pixels GP1 (identification of the stem) or in the case where the set of reference pixels GPx is equal to the corresponding second set of pixels GP2 (identification of the calyx). The perimeter of the projection of the approximant sphere (indicated by 501) and a pixel Ccx which corresponds to the aforementioned centre Cc, and therefore has the coordinates (Xc,Yc), are also visible.

Subsequently, as shown in Figure 9, the processing system 10 determines (block 208) the orientation of a segment 98, which has a length equal to the aforementioned radius and joins the main point Pp to the centre Cc of the approximant sphere. In particular, the processing system 10 calculates the angles θ and φ which define the orientation of the segment 98 in a spherical reference system centred in the centre Cc and having an axis 99 parallel to the x axis, i.e. parallel to the direction of movement of the fruits along the conveyor belt 2. The angles θ and φ therefore represent an estimate of the orientation of the fruit.

In detail:

$$\theta = arctan\left(\frac{Yp - Yc}{Xp - Xc}\right)$$

$$\varphi = arccos\left(\frac{\sqrt{(Xp - Xc)^2 + (Yp - Yc)^2}}{R}\right)$$

The advantages enabled by the present system are clearly apparent from the foregoing description. In particular, the present system allows the orientation of the fruits to be determined. Therefore, the present system can be coupled to a pick and place system 300, shown schematically in Figure 1, which is suitable to pick the fruit according to the position of the fruit, determined in a manner known per se, for example through the sensors 9, and to the orientation of the fruit.

For example, the pick and place system 300 can vary the orientation in which it picks each fruit, according to the orientation of the fruit and to the desired position and orientation inside a package in which the fruit must be placed. Furthermore, given a predefined orientation, the pick and place system 300 may be able to rotate the fruit as a function of the angles θ and φ determined during the operations referred to in block 208, so that the fruit is arranged in the package with the predefined orientation.

The present system for determining the orientation of a fruit or vegetable product also allows the orientation to be determined very quickly and without having to handle the product, thus allowing it to be integrated into existing lines in parallel with human operators. Furthermore, it can be coupled to one or more known pick and place systems. Similarly, as previously mentioned, the present orientation determination system can be coupled to any fruit singulation device, as well as to a fruit transport system other than the conveyor belt 2. For example, the fruits can be transported on corresponding supports arranged in succession and spaced apart, each bearing a single fruit.

Lastly, it is clear that the system described and illustrated herein can be subject to modifications and variations without however departing from the protective scope of the present invention, as defined in the appended claims.

For example, the number of profilometers, and therefore the number of data structures, may be different from what is described above, just like the methods of generating the aggregate data structure SDg, the filtered data structure SD_Ip1 and the processed image Ip2. For example, if there is only one profilometer, the aggregate data structure SDg coincides with the first data structure SD1. Furthermore, the processed image Ip2 may be generated as a function of the heights of a surface other than the filtered surface defined by the points of the filtered data structure SD_Ip1, this different surface being obtained, for example, from the filtered surface through a further filtering process.

More generally, instead of the profilometers, it is possible to use different types of capture devices (i.e., scanning devices, in particular of the digital type), such as for example video cameras or stereo cameras, as mentioned above.

It is also possible to determine an approximant geometric shape other than a sphere or a portion of a sphere. For example, it is possible to determine an ellipsoid or a portion thereof, in which case the Zp coordinate of the main point Pp, in addition to the coordinates (Xp,Yp), also depends on the axes of the ellipsoid.

Lastly, instead of the stem and/or the calyx, it is possible that the neural network is trained to detect other characteristic elements of the fruit or vegetable product, for example according to the type of fruit or vegetable products to be handled.

**Claims**

1.  A system for determining the orientation of a fruit or vegetable product, comprising:

    - a number of capture devices (6,8) configured to frame the fruit or vegetable product and generate corresponding data structures (SD1, SD2), each of which is indicative of the three-dimensional positions of a corresponding plurality of points of the fruit or vegetable product;
    - first processing means (10) configured to determine, based on said data structures, the three-dimensional position of the centre (Cc) of a three-dimensional geometric shape that approximates a first surface that depends on the three-dimensional positions of said points;
    - second processing means (10) configured to determine an initial image (Ip2) formed by a plurality of pixels, which are indicative of the distances from a reference plane (xy) of corresponding portions of a second surface that depends on the first surface;
    - third processing means (10) configured to implement a trained neural network so as to detect, within the initial image, the presence and the position of a first set of pixels (GP1), which forms the image of a first characteristic element of the fruit or vegetable product, and/or of a second set of pixels (GP2), which forms the image of a second characteristic element of the fruit or vegetable product;
    - fourth processing means (10) configured to determine the three-dimensional position of a reference point (Pp) based on the positions of the first and/or second set of pixels detected within the initial image; and
    - fifth processing means (10) configured to calculate a pair of angles indicative of the orientation of the fruit or vegetable product, based on the three-dimensional positions of the reference point and of the centre of said three-dimensional geometric shape.

2.  The system according to claim 1, wherein said first and second characteristic elements are respectively the calyx and the stem of the fruit or vegetable product.

3.  The system according to claim 1 or 2, wherein said three-dimensional geometric shape has a shape chosen from among: a sphere, a sphere portion, an ellipsoid and an ellipsoid portion; and wherein the fourth processing means (10) are configured to determine the three-dimensional position of the reference point (Pp) also as a function of the radius of said sphere or sphere portion, if said three-dimensional geometric shape has a spherical shape or a spherical shaped portion, or as a function of the axes of the ellipsoid, if said three-dimensional geometric shape has an ellipsoid shape or an ellipsoid shaped portion.

4.  The system according to any one of the preceding claims, wherein the first processing means (10) are configured to select the points of the data structures (SD1,SD2) that fall within a space portion and to generate a filtered data structure (SD_Ip1), which is indicative of the three-dimensional positions of corresponding points, the three-dimensional position of each of said points of the filtered data structure being the function of the three-dimensional positions of a corresponding selected point and of at least another selected point adjacent to said corresponding selected point; and wherein said first surface is defined by the three-dimensional positions of the points of the filtered data structure.

5.  The system according to any one of the preceding claims, wherein the second surface coincides with the first surface.

6.  The system according to any one of the preceding claims, wherein the neural network is configured to provide a first and a second index of confidence, respectively indicative of the probability of the correct identification of the presence of the first characteristic element inside the image formed by the first set of pixels (GP1) and of the probability of the correct identification of the presence of the second characteristic element inside the image formed by the second set of pixels (GP2); and wherein the fourth processing means (10) are configured to select, from between the first and the second set of pixels, the set of pixels (GPx) having the greatest index of confidence; and wherein the fourth processing means are configured to determine the three-dimensional position of the reference point (Pp) based on the position of the selected set of pixels, inside the initial image (Ip2).

7.  A system for handling fruit or vegetable products comprising:

    - a handling system (2) configured to move the fruit or vegetable products; and
    - a system (4,6,10) according to any one of the preceding claims, operatively coupled to the handling system.

8.  The handling system according to claim 7, wherein said number of capture devices (6,8) are configured so that the corresponding data structures (SD1,SD2) are generated while the fruit or vegetable product is moved by the handling system (2).

9. The handling system according to claim 7 or 8, wherein the handling system (2) is configured to move the fruit or vegetable products along a direction of movement (x) .

10. The handling system according to any one of the claims 7 to 9, further comprising a pick and place system (300), which is configured to individually pick the fruit or vegetable products, while they are moved by the handling system (2).

11. The handling system according to claim 10, wherein the pick and place system (300) is configured to vary the orientation in which the fruit or vegetable products are picked, according to the corresponding orientations.

**FIG. 1**

**FIG. 7**

202 — DETERMINE GP1 and/or GP2 AND THE INDEXES OF CONFIDENCE THEREOF

204 — DETERMINE GPx

206 — DETERMINE PIXEL Ppx

208 — DETERMINE ANGLES $\varphi, \vartheta$

**FIG. 2**

100 — DETERMINE THE AGGREGATE DATA STRUCTURE SDg

102 — DETERMINE THE IMAGE Ip2 AND THE FILTERED DATA STRUCTURE SD_Ip1

104 — DETERMINE THE CENTRE AND RADIUS OF THE APPROXIMANT SPHERE

106 — DETERMINE THE ORIENTATION

**FIG. 3**

FIG. 4A

FIG. 4B

FIG. 5

FIG. 6

**FIG. 8A**  **FIG. 8B**

**FIG. 9**

Europäisches
Patentamt

European
Patent Office

Office européen
des brevets

# EUROPEAN SEARCH REPORT

Application Number

EP 19 20 9590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHANG BAOHUA ET AL: "Computer vision recognition of stem and calyx in apples using near-infrared linear-array structured light and 3D reconstruction", BIOSYSTEMS ENGINEERING, ACADEMIC PRESS, UK, vol. 139, 22 August 2015 (2015-08-22), pages 25-34, XP029288004, ISSN: 1537-5110, DOI: 10.1016/J.BIOSYSTEMSENG.2015.07.011 * the whole document * | 1-11 | INV. G06T7/73 G01N21/88 G01N33/02 <br><br> ADD. G01N21/84 |
| A | Peteris Eizentals: "Picking System for Automatic Harvesting of Sweet Pepper", , September 2016 (2016-09), XP055618365, Kochi, Japan Retrieved from the Internet: URL:http://hdl.handle.net/10173/1417 [retrieved on 2019-09-04] * abstract, chapter 3, Figs. 3-3, 3-9 * | 1-11 | |
| A | WEILIN WANG ET AL: "Size estimation of sweet onions using consumer-grade RGB-depth sensor", JOURNAL OF FOOD ENGINEERING, vol. 142, December 2014 (2014-12), pages 153-162, XP055617150, GB ISSN: 0260-8774, DOI: 10.1016/j.jfoodeng.2014.06.019 * abstract, Figs. 3-4, section 2.6 * | 1-11 | TECHNICAL FIELDS SEARCHED (IPC) <br><br> G06T G01N |
| A | CN 105 158 275 A (BEIJING RES CT INTELLIGENT EQUIPMENT AGRICULTURE) 16 December 2015 (2015-12-16) * the whole document * | 1-11 | |

-/--

The present search report has been drawn up for all claims

| Place of search | Date of completion of the search | Examiner |
|---|---|---|
| Munich | 11 February 2020 | Borotschnig, Hermann |

EPO FORM 1503 03.82 (P04C01)

page 1 of 2

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | LINO ANTONI GIEFER ET AL: "Deep Learning-Based Pose Estimation of Apples for Inspection in Logistic Centers Using Single-Perspective Imaging", PROCESSES, vol. 7, no. 7, 4 July 2019 (2019-07-04), page 424, XP055618274, DOI: 10.3390/pr7070424 * the whole document * ----- | | |

TECHNICAL FIELDS
SEARCHED     (IPC)

The present search report has been drawn up for all claims

| Place of search | Date of completion of the search | Examiner |
|---|---|---|
| Munich | 11 February 2020 | Borotschnig, Hermann |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

**EP 3 654 288 A1**

## ANNEX TO THE EUROPEAN SEARCH REPORT
## ON EUROPEAN PATENT APPLICATION NO.

EP 19 20 9590

11-02-2020

| Patent document cited in search report | Publication date | Patent family member(s) | Publication date |
|---|---|---|---|
| CN 105158275 A | 16-12-2015 | CN 104463850 A<br>CN 105158275 A | 25-03-2015<br>16-12-2015 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 102018000010371

**Non-patent literature cited in the description**

- Computer vision recognition of stem and calyx in apples using near-infrared linear-array structured light and 3D reconstruction. **ZHANG BAOHUA et al.** Biosystems engineering. Academic Press, 22 August 2015, vol. 139, 25-34

- **PETERIS EIZENTALS.** *Picking system for automatic harvesting of sweet pepper,* 01 September 2016, http://hdl.handle.net/10173/1417
- **WEILIN WANG et al.** Size estimation of sweet onions using consumer-grade RGB-depth sensor. *Journal of food engineering,* December 2014, vol. 142, 153-162